# FASCICULE DE BREVET EUROPEEN

(11) **EP 3 570 923 B1**
(45) Date de publication et mention de la délivrance du brevet: **10.03.2021**
(21) Numéro de dépôt: 18700770.3
(22) Date de dépôt: 22.01.2018
(51) Int. Cl.: A61M 21/00, A63J 5/00, H04R 1/02

(54) **DISPOSITIF IMMERSIF COMPORTANT UN ECRAN ET AU MOINS DEUX CAISSONS MULTI SENSORIELS**
IMMERSIVE VORRICHTUNG MIT EINEM BILDSCHIRM UND MINDESTENS ZWEI MULTISENSORISCHEN GEHÄUSEN
IMMERSIVE DEVICE COMPRISING A SCREEN AND AT LEAST TWO MULTISENSORY CASES

(30) Priorité: 23.01.2017 FR 1754720; 23.01.2017 FR 1770070
(43) Date de publication de la demande: 27.11.2019
(73) Titulaire: The Lab In The Bag, 75011 Paris (FR)
(72) Inventeur: GACHET, Jerôme, 77940 Thoury Ferolles (FR)
(74) Mandataire: Ex Materia
(86) Numéro de dépôt international: PCT/EP2018/051462
(87) Numéro de publication internationale: WO 2018/134408

(56) Documents cités:
- WO-A1-97/37693
- WO-A1-03/089100
- WO-A1-2015/188211
- CN-U- 204 032 800
- DE-A1- 4 314 886
- US-A- 4 603 030
- US-A1- 2003 089 370
- US-A1- 2011 319 180

## Description

L'invention concerne les dispositifs immersifs, et plus particulièrement les dispositifs dans lesquels des caissons sont ménagés pour la production d'éléments sensitifs.

Un dispositif immersif peut être destiné à tester le comportement d'un utilisateur d'un produit de consommation, ou à faciliter l'évaluation ultérieure de ce produit par l'utilisateur. Les produits de consommation sont souvent évalués par des utilisateurs dans des conditions qui ne reproduisent pas l'environnement réel dans lequel ils sont utilisés. Ceci est le cas par exemple lorsque les produits sont soumis à des panels dans un laboratoire. Dans ce cas, l'utilisateur est généralement perturbé par le fait que les conditions du test ne correspondent aux conditions écologiques d'usage. L'utilisateur peut en outre être dérangé par les interactions avec les observateurs. Le comportement de l'utilisateur peut donc ne pas correspondre au comportement qu'il aurait dans un environnement réel.

Un dispositif immersif permet d'améliorer le test comportemental ou l'évaluation marketing en permettant à l'utilisateur de tester le produit de consommation dans un environnement approprié.

A titre d'exemple non limitatif, on peut proposer de tester le parfum d'une crème solaire dans un dispositif immersif où des images et un son de plage sont projetés, ou de tester le goût d'un aliment dans un dispositif immersif où sont plutôt projetés des images et un son de salle de restaurant.

Les dispositifs immersifs connus combinent la projection d'images et de sons autour de l'utilisateur. Un ou plusieurs écrans sont prévus et des enceintes génératrices de son sont répartis en plusieurs points de la salle dans laquelle est installé le dispositif, ce qui permet de créer des effets sonores réalistes, c'est-à-dire en adéquation avec ce qui est projeté sur le ou sur les écrans.

Ces dispositifs immersifs peuvent également être mis en oeuvre pour des applications de loisir et de divertissements audiovisuels, et dans ces applications la demande des utilisateurs est de plus en plus exigeante.

Or, dans les dispositifs connus, l'immersion de l'utilisateur n'est pas totale, puisqu'elle concerne uniquement deux sens que sont la vue et l'ouïe.

On connait par ailleurs des dispositifs immersifs dans lesquels on génère un effet sensoriel additionnel autre que le son, et notamment du vent ou des odeurs. Un problème des dispositifs connus est que la source d'émission de l'effet sensoriel additionnel est ponctuelle ce qui rend peu efficace l'immersion sensorielle.

Or, il est recherché l'immersion la plus totale quelle que soit l'application souhaitée pour ces dispositifs immersifs.

Dans ce contexte, l'invention vise à proposer une alternative aux équipements existants pour réaliser ces dispositifs immersifs et de façon correspondante une alternative aux dispositifs immersifs connus.

L'invention propose à cet effet un dispositif immersif comportant un écran et une pluralité de caissons multi sensoriels qui délimite avec l'écran une zone immersive, les différents caissons multi sensoriels étant disposés sur la périphérie de la zone immersive, chaque caisson multi sensoriels comportant au moins un dispositif de génération d'un flux d'air et un dispositif générateur de son, ainsi qu'un dispositif de contrôle qui est configuré pour gérer l'émission de son et d'air par un pilotage indépendant de chacun des caissons multi sensoriels.

En d'autres termes, l'invention propose un dispositif immersif comportant un écran, qui peut être courbe, et une pluralité de caissons multisensoriels qui délimite avec l'écran une zone immersive, les différents caissons multisensoriels étant disposés sur la périphérie de la zone immersive, chaque caisson multisensoriel comportant au moins un dispositif de génération d'un flux d'air et un dispositif générateur de son, ainsi qu'un dispositif de contrôle qui est configuré pour gérer l'émission de son et d'air par un pilotage indépendant de chacun des caissons multisensoriels. Par périphérie, on comprend que les caissons multisensoriels bordent la zone immersive, et ce aussi bien sur le côté qu'au-dessus de la zone immersive dans laquelle est amené à se déplacer l'utilisateur.

Au moins un caisson multi sensoriel peut comporter au moins un dispositif de diffusion d'odeur, formé d'au moins un support de diffusion d'odeur et un ventilateur, et un dispositif de génération d'un flux d'air, le dispositif de diffusion d'odeur et le dispositif de génération d'un flux d'air étant disposés l'un au-dessus de l'autre.

Selon différentes caractéristiques du dispositif immersif selon l'invention, prises seules ou en combinaison, on pourra prévoir que :
- le dispositif immersif comporte un caisson central agencé entre les au moins deux caissons multi sensoriels ;
- le dispositif immersif comporte un support d'écran et des caissons multi sensoriels disposés de part et d'autre de l'écran, le support comportant une barre agencée entre les deux caissons pour porter le caisson central ;
- le caisson central comporte un ventilateur configuré pour redistribuer l'air brassé par les dispositifs de génération d'un flux d'air agencés dans chaque caisson multi sensoriel ;
- l'écran est courbe ; par écran courbe, on entend couvrir aussi bien le fait que la courbure de l'écran est régulière que le fait que la courbure de l'écran est complexe, à facettes ; par extension, il pourrait être prévu que l'écran soit composé d'une pluralité de portions d'écrans plats inclinés angulairement les uns par rapport aux autres de manière à reproduire un ensemble globalement courbe ;
- les au moins deux caissons multi sensoriels comportent deux caissons multi sensoriels latéraux agencés respectivement dans le prolongement des extrémités de l'écran courbe ;
- le dispositif immersif comporte une pluralité de caissons multi sensoriels qui délimite avec l'écran une zone immersive ;
- le caisson central est disposé au centre et au-dessus de la zone immersive ; par « au centre » de la zone immersive, on comprend que la largeur de la zone immersive est définie par l'écartement entre deux caissons latéraux disposés au voisinage de l'écran, et que le caisson central est disposé sur la médiatrice entre ces deux caissons latéraux ; et par « au-dessus », on comprend que le caisson central surplombe l'usager installé dans la zone immersive ;
- l'écran est associé à un dispositif de projection d'images ; et le dispositif de projection d'images est un rétroprojecteur agencé du côté de l'écran opposé à la zone immersive ;
- le dispositif immersif comporte deux caissons multi sensoriels dit avants, agencés au voisinage direct de l'écran, deux caissons multi sensoriels dit arrière, agencés à l'extrémité opposée de la zone immersive, et un caisson central ;
- le module de commande, ou dispositif de contrôle, est configuré pour répartir de façon équilibrée les effets entre chacun des caissons multi sensoriels en fonction de l'image sur l'écran.

D'autres caractéristiques et avantages de la présente invention apparaitront plus clairement à l'aide de la description et des dessins parmi lesquels :
- la figure 1 est une vue d'ensemble, en perspective, d'un dispositif immersif équipé d'une pluralité de caissons multi sensoriels et d'un écran délimitant une zone immersive, la perspective rendant par ailleurs visible un dispositif de rétroprojection configuré pour émettre des images sur l'écran ;
- la figure 2 est une vue de dessus du dispositif immersif de la figure 1 ;
- la figure 3 est une vue, de détail d'un caisson multi sensoriel équipant le dispositif immersif de la figure 1 ;
- la figure 4 est une représentation schématique, vue de face, de deux caissons multi sensoriels d'un premier type tel qu'illustré sur la figure 3 ;
- la figure 5 est une vue partielle, en coupe selon le plan A-A de la figure 4, d'un caisson multi sensoriel d'un premier type ;
- la figure 6 est une représentation schématique, vue de face, d'un caisson multi sensoriel d'un deuxième type ;
- la figure 7 est une représentation schématique du dispositif immersif illustrant le pilotage des dispositifs sensoriels équipant les caissons multi sensoriels via une pluralité de modules de commande associés ; et
- la figure 8 est une représentation schématique d'une variante de réalisation du dispositif immersif selon l'invention.

Il faut tout d'abord noter que les figures exposent l'invention de manière détaillée pour mettre en oeuvre l'invention, lesdites figures pouvant bien entendu servir à mieux définir l'invention le cas échéant. Toutefois, il est à noter que ces figures n'exposent qu'une partie des variantes de réalisation possibles selon l'invention.

Dans la description qui va suivre, on se référera à une orientation en fonction des axes longitudinaux L, verticaux V et transversaux T, tels qu'ils sont définis arbitrairement par le trièdre L,V,T représenté sur les figures. Notamment, l'axe vertical V correspond à la direction perpendiculaire au sol, de sorte que l'on définira les positions au-dessous ou au-dessous en fonction de la verticalité le long de cet axe vertical V.

Un dispositif immersif 1 selon un aspect de l'invention comporte au moins un écran 2, servant de support de projection d'une image ou d'une vidéo, et au moins deux caissons multi sensoriels 4 configurés pour émettre plusieurs effets sensoriels en fonction de l'image projeté simultanément sur l'écran. L'objectif d'un tel dispositif est de proposer une expérience d'immersion à un utilisateur, que ce soit dans un contexte ludique, professionnel ou encore médical. Afin de profiter au mieux de l'expérience sensitive, l'utilisateur est en situation assise ou en situation debout dans une zone immersive 6 définie par la position des caissons multi sensoriels 4, avantageusement devant et derrière lui et sur chacun de ses côtés, et par la position de l'écran 2. La pluralité de caissons multi sensoriels délimite avec l'écran la dimension et la forme de cette zone immersive, et donc de façon indirecte le nombre d'utilisateurs pouvant utiliser simultanément cette zone immersive.

Dans l'exemple illustré, le dispositif immersif 1 comporte au moins quatre caissons multi sensoriels 4 selon un aspect de l'invention, à savoir des caissons de type colonne dans lequel des dispositifs sensoriels 8 embarqués dans ce caisson sont empilés verticalement les uns au-dessus des autres. On saura décrire ci-après des agencements spécifiques des dispositifs sensoriels dans ces caissons de type colonne.

Parmi les quatre caissons multi sensoriels, on distingue deux premiers caissons multi sensoriels, dit caissons multi sensoriels avants 4-AV, agencés au voisinage direct de l'écran 2, deux deuxièmes caissons multi sensoriels, dits caissons multi sensoriels arrières 4-AR et qui sont agencés à l'extrémité opposée de la zone immersive 6 par rapport à l'écran 2 et aux premiers caissons multi sensoriels, et un caisson central 10, disposé au voisinage de l'écran 2 et entre les deux caissons multi sensoriels avants 4-AV.

Le dispositif immersif comporte en outre au moins un dispositif de contrôle pour le pilotage des caissons multi sensoriels 4, qui comprend avantageusement à cet effet un module de commande principal 12, et une pluralité de modules de commande spécifiques 14, dédiés à chacun des caissons multi sensoriels 4.

Le module de commande principal 12 est configuré pour définir, pour un instant défini de la vidéo projeté sur l'écran, une pluralité d'instructions de commande respectivement dédiées à l'un et/ou l'autre des dispositifs sensoriels, et à l'un et/ou l'autre des caissons multi sensoriels. Les modules de commande, principal ou spécifique, sont configurés pour gérer l'émission des éléments sensoriels par un pilotage indépendant de chacun des caissons, de manière à répartir de façon équilibrée les effets sensoriels entre chacun des caissons multi sensoriels en fonction de l'image sur l'écran.

On comprend que par répartir de façon équilibrée les effets entre chacun des caissons, il est possible qu'en fonction d'une première image sur l'écran chacun des caissons soit piloté pour réaliser la même émission sensorielle dans les mêmes proportions, et qu'en fonction d'une deuxième image, seul l'un des caissons soit piloté pour réaliser une émission sensorielle tandis que les autres restent inactifs.

Tel que cela sera décrit ci-après, un caisson multi sensoriel 4 selon l'invention peut comporter une pluralité de dispositifs sensoriels 8 empilés les uns au-dessus des autres, afin de réaliser des effets sensoriels variés pour que l'utilisateur ait au moins une sensation auditive, olfactive, ou haptique en plus de la sensation visuelle assurée par la projection d'images sur l'écran.

L'écran 2 peut notamment consister en un support de projection d'images, associé à un dispositif de projection d'images 16, par exemple de type vidéoprojecteur ou rétroprojecteur.

Tel qu'illustré, l'écran est associé à un dispositif de projection d'image de type rétroprojecteur. De la sorte, le dispositif de projection d'image 16 est agencé derrière l'écran et il n'est pas présent dans la zone immersive. Il en résulte une meilleure immersion pour l'utilisateur qui d'une part ne voit pas le dispositif formant source des images projetées sur l'écran et qui d'autre part entend peu ou pas le souffle continu du au fonctionnement du projecteur.

Tel qu'illustré, le rétroprojecteur peut être associé à un miroir 18 pour améliorer la compacité du dispositif immersif 1. Le rétroprojecteur émet un faisceau lumineux à l'opposé de l'écran, et ce faisceau est réfléchi par le miroir en direction de la face arrière de l'écran 2, c'est-à-dire la face opposée à la zone immersive 6. Le rétroprojecteur est alors agencé entre l'écran 2 et le miroir 18 et il n'est pas nécessaire d'avoir un recul trop important pour que l'image projetée s'étale sur tout l'écran.

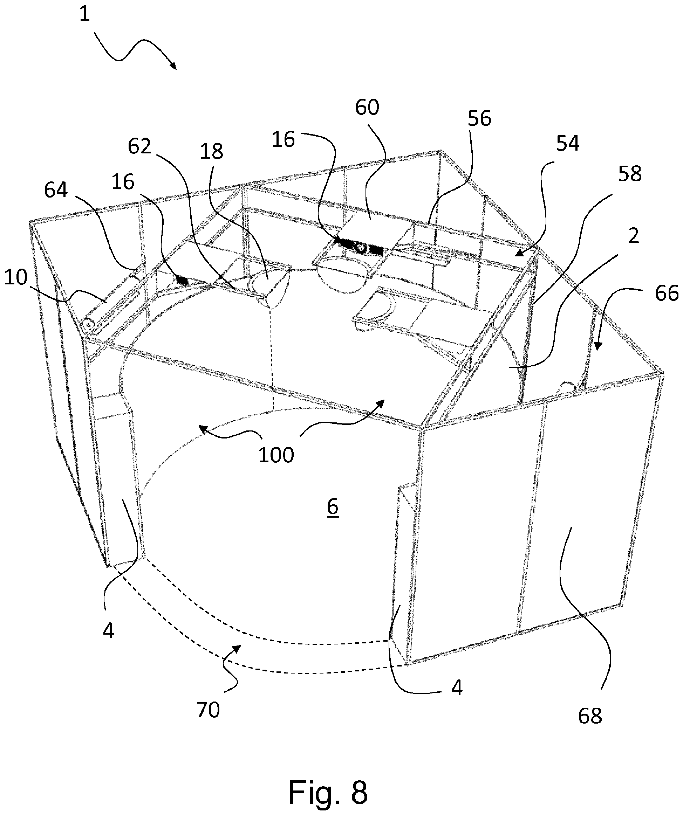

Dans une variante de réalisation illustrée en figure 8 et qui sera décrit ci-après, le dispositif de projection d'image peut consister en un vidéoprojecteur agencé au-dessus de l'écran, au moins en partie au-dessus de la zone immersive. Cet agencement permet de projeter l'image sur l'écran sans que la présence d'utilisateur au centre de la zone immersive ne gêne la projection d'images, et permet de limiter l'encombrement, ou de dégager des zones pour l'électronique de commande notamment, derrière l'écran.

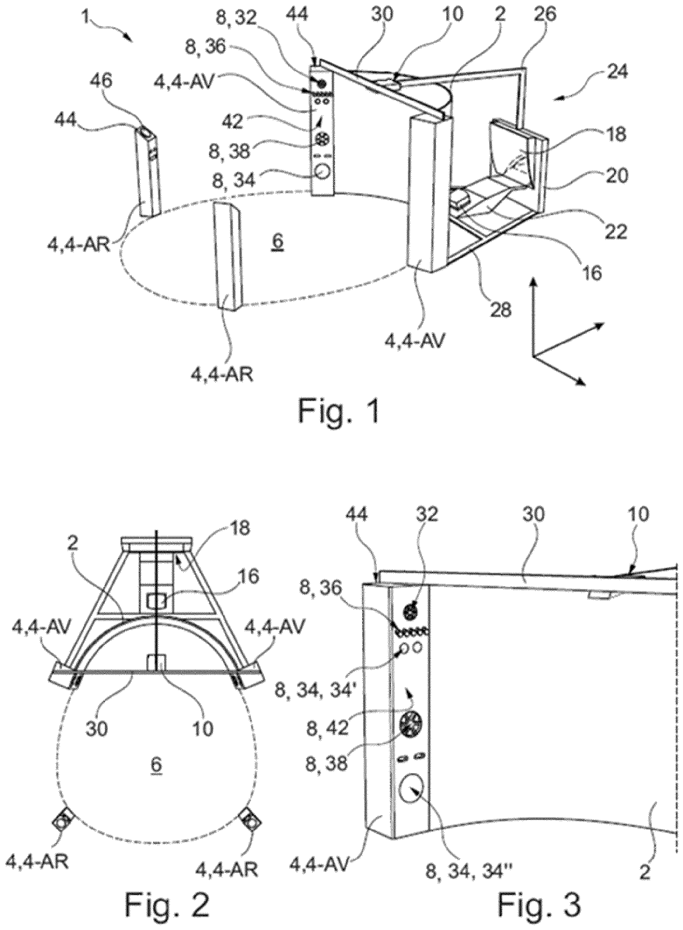

Dans ce contexte, la position du miroir 18 est définie par rapport à l'écran 2, puis la position du projecteur 16 est définie par rapport au miroir 18. Le miroir est fixé sur une première platine 20 et le projecteur est lui fixé sur une deuxième platine 22, les deux platines pouvant être parties d'une même pièce, de sorte que la position relative du projecteur par rapport au miroir est déjà connue et qu'il ne reste plus qu'à régler la position du miroir par rapport à l'écran. Un support 24, visible sur les figures 1 et 2, peut faciliter la fixation du miroir par rapport à l'écran, pour assurer une position fiable et donc une image nette à chaque installation du dispositif immersif, sans qu'il soit nécessaire de régler à chaque installation la distorsion de l'image.

Le support comporte notamment une potence 26 reliant le centre de l'écran 2, et le cas échéant la partie supérieure d'un châssis supportant l'écran, à la première platine 20 porteuse du miroir. On définit de la sorte la position centrée transversalement du miroir, et par conséquent du projecteur, par rapport à l'écran, et on s'assure de la mise en oeuvre de l'écartement entre le miroir et l'écran tel qu'il a été défini théoriquement en fonction du réglage de la distorsion établi au préalable.

Le support peut comporter en outre une base 28, amenée à être posée au sol et qui permet de relier la première platine 20 porteuse du miroir d'une part aux caissons multi sensoriels avants 4-AV et d'autre à une partie inférieure du châssis supportant l'écran. On assure ainsi simultanément la bonne position relative de l'écran et du miroir faisant partie du système de projection, pour la netteté des images, ainsi que la bonne position des caissons multi sensoriels par rapport aux images projetées.

Tel que cela vient d'être précisé, l'écran peut être porté par un châssis, qui prend une forme courbe permettant de conformer l'écran. Il peut en effet être intéressant, afin d'augmenter l'effet immersif pour l'utilisateur, de le mettre en condition dans une zone immersive délimitée par un écran courbe. Le châssis comporte une partie supérieure et une partie inférieure sur lesquelles sont tendus les bords supérieur et inférieur de l'écran 2.

Ces parties supérieure et inférieure du châssis peuvent être fixées à chacune de leurs extrémités latérales à un portique vertical délimitant le bord latéral de l'écran, et il sera compris que dans l'exemple illustré, sans que cela soit limitatif de l'invention, les parties supérieure et inférieure du châssis d'écran sont respectivement fixées, notamment par un système de vissage quart de tour, sur les caissons multi sensoriels avants 4-AV disposés de part et d'autre de l'écran 2.

Tel qu'illustré sur la figure 2, les deux caissons multi sensoriels avants 4-AV disposés de part et d'autre de l'écran sont agencés respectivement dans le prolongement des extrémités de l'écran 2 ici courbe.

L'écran est courbe notamment pour que toute la surface de cet écran soit à une distance équivalente de l'utilisateur placé au centre de la zone immersive, ce qui minimise la fatigue oculaire de l'utilisateur et maximise son confort de vision et donc son immersion.

Le support peut comporter une barre 30 agencée entre les deux caissons multi sensoriels avant 4-AV, et qui repose à ses extrémités transversales sur le sommet des caissons, de manière à s'étendre au-dessus de l'écran. Cette barre du support est configurée pour porter, sensiblement en son centre, un boitier support dans lequel le caisson central 10 est agencé. De la sorte, le caisson central 10 est disposé au centre et au-dessus de la zone immersive 6.

Dans la variante de réalisation évoquée précédemment, le vidéo projecteur, formant le dispositif de projection 16 apte à projeter une image du côté de l'écran correspondant à la zone immersive, peut être montée sur cette barre 30. Et il convient de noter que le fait que la barre 30 repose sur les extrémités transversales sur le sommet des caissons pourrait être modifié sans sortir du contexte de l'invention, notamment par la mise en place d'un portique qui sera décrit ci-après plus en détails.

Les différents caissons multi sensoriels 4 sont disposés sur la périphérie de la zone immersive et il convient de noter que chaque caisson comporte au moins un dispositif générateur de flux d'air 32 et un dispositif générateur de son 34, dans le contexte de dispositifs sensoriels 8 empilés les uns sur les autres dans un caisson de type colonne.

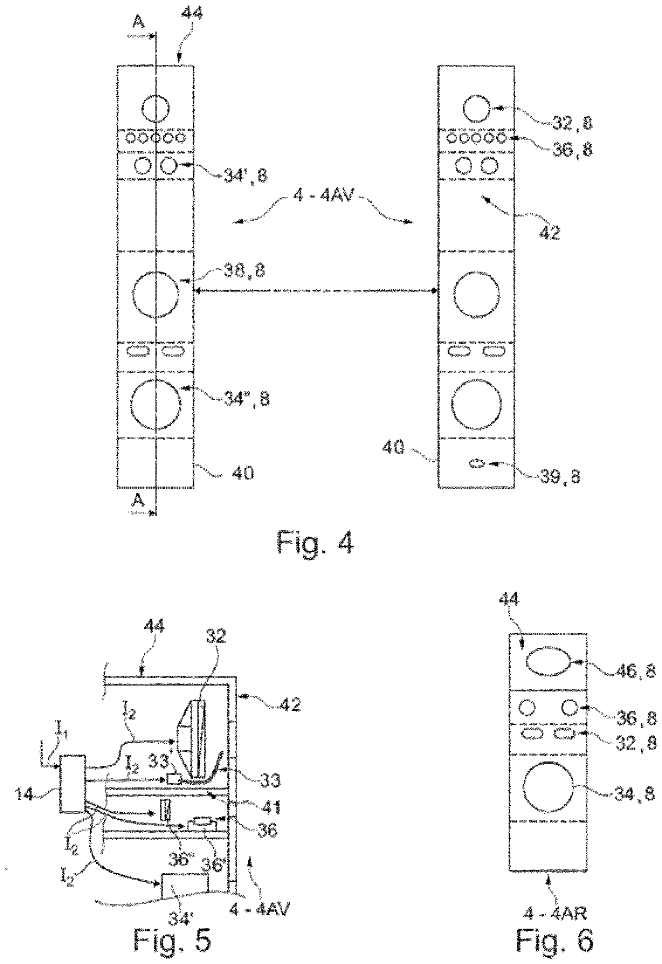

Dans l'exemple de réalisation présentement décrit et notamment illustré sur la figure 5, il convient de noter que chacun des caissons multi sensoriels 4 comporte, dans le contexte de dispositifs sensoriels empilés les uns sur les autres dans un caisson de type colonne, au moins un dispositif de génération d'un flux d'air 32, un dispositif de génération de son 34, ainsi qu'un dispositif de diffusion d'odeur 36, et que le caisson central 10 comporte au moins ces mêmes dispositifs sensoriels.

Le caisson central 10 comporte notamment un ventilateur central 10' configuré pour redistribuer l'air brassé par les dispositifs de génération d'un flux d'air 32 agencés dans les caissons multi sensoriel avants 4-AV, disposés de part et d'autre de l'écran 2. Par exemple, les dispositifs de génération d'un flux d'air 32 agencés dans chaque caisson multi sensoriel avant sont respectivement orientés vers le caisson central.

Tel que cela sera décrit plus en détails ci-après, on peut en effet noter que chacun des dispositifs sensoriels peut être agencé au sein d'un caisson multi sensoriel 4 de manière à émettre dans une direction donnée, qui peut être différente de la direction dans laquelle émet un autre dispositif sensoriel du même caisson, ou bien différente de la direction dans laquelle émet un dispositif sensoriel équivalent d'un autre caisson. A titre d'exemple, les dispositifs de génération de son de chacun des caissons multi sensoriels seront agencés pour le son soit émis vers le centre de la zone immersive, tandis que le flux d'air et les odeurs émis par les caissons multi sensoriels avant seront dirigés vers le caisson central pour être ensuite brassés via le ventilateur de ce caisson central.

Une toile peut être tendue dans le prolongement de l'écran pour masquer la jonction entre l'écran et les caissons multi sensoriels avants, c'est-à-dire les caissons les plus susceptibles d'être perçus par l'utilisateur regardant l'écran. Cette toile peut habiller le caisson, au moins sur les zones dans lesquelles il n'y a pas de sorties d'un dispositif sensoriel, d'une teinte proche de ce qui est projeté sur l'écran.

On pourra en outre prévoir des moyens de projection additionnels permettant la réalisation de cartographie vidéo, également connu sous la dénomination anglophone de mapping video, par l'intermédiaire desquels on projette un fond d'images complémentaires des images projetées sur l'écran.

On va maintenant décrire plus en détails un caisson multi sensoriel 4 formant partie du dispositif immersif selon l'invention, en se référant notamment aux figures 3 à 6.

Un caisson multi sensoriel 4 comporte un bâti 40 formant support des dispositifs sensoriels. Le bâti présente la forme générale d'une colonne, dans le cas illustré de section carrée, et ce bâti comporte une pluralité de supports 41 de positionnement des dispositifs sensoriels 8. A titre d'exemple, et tel que cela est illustré sur la figure 5, le caisson présente la forme d'une étagère avec plusieurs tablettes formant support 41 superposées et sur lesquelles sont positionnées les dispositifs sensoriels 8 et les modules de commande spécifiques 14 associés.

On peut définir une face avant 42 du bâti comme la face destinée à être tournée vers le centre de la zone immersive, lorsque l'ensemble des caissons est mis en place autour ou à proximité de l'écran. Les dispositifs sensoriels sont configurés pour déboucher et émettre principalement sur une même face du bâti, à savoir cette face avant du bâti. Toutefois, on pourrait comprendre que sans sortir du contexte de l'invention, un ou plusieurs dispositifs sensoriels pourraient avoir une sortie ménagée dans une face directement adjacente de cette face avant.

Chaque caisson selon aspect de l'invention comporte une pluralité de dispositifs sensoriels 8, générateurs d'effets sensoriels distinctifs, disposés verticalement les uns au-dessus des autres, de manière à former un caisson de type colonne.

De préférence, les dimensions du bâti 40 pour former un caisson de type colonne destiné à être implanté à l'avant ou à l'arrière de la zone immersive 6 sont sensiblement les mêmes pour ce qui est de la définition de la base du caisson, et par exemple de l'ordre de 50 cm pour sa largeur et pour sa profondeur, et varient pour définit la hauteur du caisson. La hauteur peut ainsi être égale à 2,10 m pour les caissons multi sensoriels avants 4-AV, et égale à 1,4 m pour les caissons multi sensoriels arrières 4-AR.

Tel que cela sera décrit ci-après, les caissons multi sensoriels avant 4-AV et arrière 4-AR peuvent différer, outre par leur hauteur, par la forme de leur paroi supérieure 44, avec une paroi parallèle au sol pour les caissons multi sensoriels avants et une paroi inclinée par rapport au sol pour les caissons multi sensoriels arrières.

Dans un premier temps, on va se référer notamment aux figures 3 à 5 pour décrire un caisson multi sensoriel avant 4-AV.

A titre d'exemple illustré, un caisson multi sensoriel peut comporter, de haut en bas, un dispositif de génération d'un flux d'air 32, un dispositif de diffusion d'odeur 36, un dispositif générateur de sons aigus 34', un dispositif de génération d'un flux d'air additionnel 38 et un dispositif générateur de sons à basse fréquence 34".

Le dispositif de diffusion d'odeur 36 est formé d'au moins un support de diffusion d'odeur 36' et un ventilateur 36", tel que visible sur la figure 5. Dans l'exemple illustré, on prévoit d'équiper les caissons multi sensoriels avants 4-AV (figure 4) de cinq dispositifs de diffusion d'odeur 36, et d'équiper les caissons multi sensoriels arrière 4-AR (figure 6) de deux dispositifs de diffusion d'odeur 36.

Pour chacun des dispositifs de diffusion d'odeur 36, le support de diffusion d'odeur 36' consiste en un réservoir contenant une substance parfumée, le réservoir pouvant être ouvert ou fermé au moyen d'une électrovanne. On comprend que lorsque le réservoir est ouvert, le parfum qui s'en exhale est mis en mouvement par le ventilateur.

On distingue dans le caisson multi sensoriel le ventilateur 36", spécifiquement dédié à un dispositif de diffusion d'odeur 36 et à un support de diffusion 36', et le dispositif de génération d'un flux d'air 32, qui est lui agencé à part du ou des dispositif(s) de diffusion d'odeur 36. Le ventilateur 36" associé au support de diffusion est déclenché uniquement lorsque le réservoir est ouvert et libère une substance parfumée. Le ventilateur est ainsi ménagé en bout d'un conduit 37 dans lequel est libéré la substance parfumée, et le ventilateur 36" et le réservoir du support de diffusion 36' sont disposés sensiblement dans un même plan horizontal, et selon l'invention à un même étage 41 du caisson multi sensoriel de type colonne. Le dispositif de génération d'un flux d'air 32 est lui agencé dans un plan vertical horizontal différent de celui dans lequel sont agencés les supports de diffusion 36' et les ventilateurs 36" associés, et il peut être déclenché indifféremment du déclenchement des dispositifs de diffusion d'odeur 36.

Tel que cela a été précédemment écrit, le caisson multi sensoriel 4 est ici équipé d'une pluralité de dispositifs de diffusion d'odeur 36, chaque dispositif de diffusion d'odeur comportant un ventilateur 36" spécifique, et le dispositif de génération d'un flux d'air 32 est commun à l'ensemble des dispositifs de diffusion d'odeur. Avantageusement, les substances parfumées contenues respectivement dans chacun des dispositifs de diffusion d'odeur sont différentes l'une de l'autre, et l'on pilote la diffusion d'une première ou d'une n-ième odeur, en fonction de l'image projetée et de l'odeur que l'on veut associer, par une ouverture d'un premier ou d'un n-ième réservoir et l'activation simultané d'un premier ou d'un n-ième ventilateur, ainsi que par l'activation du dispositif de diffusion d'odeur disposé au-dessus des dispositifs de diffusion d'odeur.

Il est en effet notable que, dans chacun des caissons multi sensoriels 4 illustrés sur les figures, le dispositif de diffusion d'odeur 36 et le dispositif de génération d'un flux d'air 32 sont disposés l'un au-dessus de l'autre. Et notamment, le dispositif de génération d'un flux d'air est disposé au-dessus du dispositif de diffusion d'odeur. Plus précisément, alors qu'un caisson multi sensoriel 4 selon un aspect de l'invention comporte une pluralité de dispositifs sensoriels parmi lesquels au moins un distinct du dispositif de génération d'un flux d'air et du dispositif de diffusion d'odeur tels qu'ils viennent d'être décrits, ces dispositifs sensoriels 8 sont agencés de sorte que le dispositif de génération d'un flux d'air 32 est directement au-dessus du dispositif de diffusion d'odeur 36.

Cet agencement permet de pousser vers le centre de la zone immersive 6, par l'effet du dispositif de génération d'un flux d'air, la substance parfumée mise en mouvement par le ventilateur et montant en sortie de la face avant 42 du caisson. Et il permet de disposer dans ce contexte le dispositif de génération d'un flux d'air 32 de chaque caisson multi sensoriel au-dessus de la tête des utilisateurs, de sorte que le flux d'air et la substance parfumée qu'il entraîne avec lui passe au-dessus des utilisateurs, rendant plus diffuse la sensation olfactive et moins agressive.

On comprend que par ailleurs, le dispositif de génération d'un flux d'air 32 peut être activé sans que soit activé le dispositif de diffusion d'odeur 36, uniquement pour la réalisation d'un effet sensoriel illustrant la présence d'air, de vent, sans odeurs spécifiques.

Les caissons multi sensoriels avant et arrière peuvent ensuite différer dans le nombre et l'agencement des dispositifs sensoriels qu'ils comportent.

Le caisson multi sensoriel avant 4-AV comporte un dispositif de génération d'un flux d'air additionnel 38, servant notamment à produire un flux d'air complémentaire lorsque les images sur l'écran nécessitent, pour optimiser l'immersion de l'utilisateur, un flux d'air important que le dispositif de génération d'un flux d'air agencé au-dessus du dispositif de diffusion d'odeur ne peut mettre en oeuvre seul. Ce dispositif de génération d'un flux d'air additionnel 38 est disposé en partie basse du caisson de type colonne, et peut le cas échéant être utilisé lorsque l'immersion nécessite la création d'un souffle rasant le sol.

Le dispositif de génération d'un flux d'air additionnel 38 est dédié uniquement à la génération d'un flux d'air pour reproduire l'effet du vent, tandis que, tel que cela a été précisé précédemment, le dispositif de génération d'un flux d'air 32 disposé au voisinage direct du dispositif de diffusion d'odeur 36 est bifonctionnel en ce qu'il permet de diffuser les odeurs générées et en ce qu'il permet de participer à la reproduction de l'effet du vent. Le dispositif de génération d'un flux d'air 32 est principalement mis en oeuvre, et le dispositif de génération d'un flux d'air additionnel 38 est uniquement mis en oeuvre quand le besoin en apport d'air est plus important que ce que peut délivrer le dispositif de génération d'un flux d'air.

La présence d'un dispositif de génération d'un flux d'air 32 et d'un dispositif de génération d'un flux d'air additionnel 38 permet, outre la possibilité offerte de quantifier la force du flux d'air généré au global, de qualifier différemment les flux d'air en fonction des besoins immersifs.

A titre d'exemple, un ou plusieurs brumisateurs 33 haute pression sont associés au dispositif de génération d'un flux d'air 32, et un dispositif de traitement thermique est associé au dispositif de génération d'un flux d'air additionnel 38 de manière à chauffer ou refroidir l'air amené à passer à travers ce dispositif de génération d'un flux d'air additionnel. Il est alors possible d'une part de réaliser un effet haptique par projection de gouttelettes sur la peau de l'utilisateur, par la mise en marche du dispositif de génération d'un flux d'air et du ou des brumisateurs dont les bouches sont disposées au plus près des pales de ce dispositif de génération d'un flux d'air, et d'autre part de réaliser un autre effet haptique par une sensation de chaleur ou de froid en activant le dispositif de génération d'un flux d'air additionnel et son dispositif de traitement thermique associé. On comprend que dans chacun de ces cas, le module de commande spécifique 14 au caisson multi sensoriel est configuré pour envoyer une instruction d'activation de chacun des composants distinctement, de manière à pouvoir déclencher le dispositif de génération d'un flux d'air 32 avec ou sans la mise en marche simultanée du brumisateur, et pour pouvoir déclencher le dispositif de génération d'un flux d'air additionnel 36 avec ou sans la mise en marche simultanée du dispositif de traitement thermique de l'air.

Le ou les brumisateurs 33 peuvent consister en des buses de projection à haute pression, par exemple à 15 bars, d'un liquide provenant d'une source 33' de stockage de liquide, agencée à proximité des buses. Ces buses sont disposées au plus près de la face avant 42 du bâti de sorte que le liquide sortant des buses est immédiatement projeté sous forme de gouttelettes sous l'effet du flux d'air soufflé par le dispositif de génération d'un flux d'air 32.

Le dispositif de traitement thermique associé au dispositif de génération d'un flux d'air additionnel 38 peut consister en un système de chauffage électrique, éventuellement avec une résistance moulée dans la céramique, et/ou en un climatiseur.

Les caissons multi sensoriels avants 4-AV comportent également un dispositif de génération de son 34 spécifique en ce qu'il comprend une pluralité des haut-parleurs disposés les uns à distance des autres, et notamment un dispositif générateur de sons aigus 34' et un dispositif générateur de sons à basse fréquence 34".

Faisant suite à ce qui précède, les dispositifs sensoriels 8 sont agencés de sorte que le dispositif générateur de sons à basse fréquence 34" est disposé sous le dispositif de génération d'un flux d'air additionnel 38, qui est lui disposé sous le dispositif générateur de sons aigus 34'. Et le dispositif générateur de sons aigus est disposé à peu près à hauteur d'homme, étant entendu que l'on pourra se fier pour cela à la taille moyenne des hommes et des femmes dans le pays d'utilisation du dispositif immersif, qui est à titre d'exemple pour la France sensiblement égale à 1,68m.

Les parties du caisson regroupant les dispositifs générateurs de sons, aigus ou à basse fréquence, peuvent est recouvertes par un tissu, de manière à rendre invisibles ces dispositifs par l'utilisateur, sans que cela gêne la propagation du son.

Par ailleurs dans ce contexte de camouflage des caissons multi sensoriels, on pourra prévoir que les bouches de ventilation des dispositifs de génération d'un flux d'air 32 et de flux d'air additionnel 38 peuvent être fermées par des systèmes amovibles, afin que ces dispositifs ne soient pas visibles lorsque le système n'est pas en fonctionnement.

Sur au moins l'un des caissons multi sensoriels, et notamment, dans le cas illustré en figure 4, sur un des caissons multi sensoriels avants, on peut par ailleurs prévoir un générateur de fumée 39 dont l'activation est également contrôlée par un module de commande spécifique au caisson.

Dans un deuxième temps, on va se référer à la figure 6 pour décrire un caisson multi sensoriel arrière. Tel que cela a été précisé précédemment et compréhensible par la comparaison schématique des figures 4 et 6, le caisson multi sensoriel arrière se distingue notamment par sa taille et le nombre de dispositifs sensoriels qu'il comporte dans son bâti.

Le bâti présente une forme différente en ce que la paroi supérieure 44, tel que cela est visible sur la figure 3, n'est pas parallèle au plan du sol mais inclinée, de sorte que la face avant destiné à être tournée vers la zone immersive et par laquelle débouchent les dispositifs sensoriels est moins haute que la face arrière opposée.

Le caisson multi sensoriel arrière diffère en outre en ce qu'il comporte un dispositif générateur de lumière 46, configuré de manière à émettre un faisceau lumineux au-dessus du caisson multi sensoriel, et donc au-dessus de l'utilisateur. On peut ainsi projeter sur un plafond de la salle dans laquelle est disposé le dispositif immersif, ou bien sur une toile tendue au-dessus de la zone immersive, une lumière d'ambiance pouvant évoluer en fonction de l'image projetée sur l'écran.

Le dispositif de génération de lumière 46 consiste en un dispositif d'éclairage multicolore, qui peut notamment être constitué d'une multitude de diodes électroluminescentes. Ainsi, la couleur dominante des différentes scènes de la vidéo projeté sur l'écran est projetée sous forme lumineuse.

On peut également noter comme différence le fait que seul un dispositif générateur de sons à basse fréquence 34" est prévu dans les caissons multi sensoriels.

Par contre, et conformément à ce qui a été décrit pour les caissons multi sensoriels avants, les caissons multi sensoriels arrières 4-AR comportent un dispositif de génération d'un flux d'air 32 et un dispositif de diffusion d'odeur 36 empilés l'un directement au-dessus de l'autre, c'est-à-dire sans dispositifs sensoriels interposés entre eux, et ce dans un caisson de type colonne, avec l'ensemble des dispositifs sensoriels empilés. Contrairement à ce qui est proposé dans les caissons multi sensoriels avants, et du fait de la hauteur à laquelle peuvent être agencés le ou les dispositifs de diffusion d'odeur dans un caisson multi sensoriel arrière, le dispositif de génération d'un flux d'air est ici disposé au-dessous du ou des dispositifs de diffusion d'odeur afin de faciliter la perception des odeurs par l'utilisateur.

Le dispositif immersif selon l'invention comporte de la sorte des caissons pour réaliser l'émission multi points, c'est-à-dire de façon simultanée depuis plusieurs endroits autour de l'utilisateur présent dans la zone immersive, de différents effets sensoriels de façon synchronisée avec la diffusion d'images sur un écran participant à délimiter cette zone immersive.

Selon le présent exemple, visible notamment sur la figure 2, on peut prévoir un premier groupe de deux caissons multi sensoriels avants 4-AV, présentant une même première hauteur, et un deuxième groupe de deux caissons multi sensoriels arrières 4-AR, présentant une même deuxième hauteur. Il est important que pour un même groupe de caissons multi sensoriels, les dispositifs sensoriels soient à même hauteur pour une homogénéité du son et des autres éléments sensoriels. En d'autres termes, il importe que les dispositifs de diffusion d'odeur disposés dans un caisson multi sensoriel à gauche de l'écran soient situés à même hauteur par rapport au sol que les dispositifs de diffusion d'odeur disposés dans un caisson multi sensoriel à droite de l'écran.

Les caissons multi sensoriels arrières peuvent être plus petits, et comprendre moins de dispositifs sensoriels que dans les caissons multi sensoriels avants, mais selon l'invention, il importe que chaque caisson présente une forme de colonne avec les dispositifs sensoriels qu'il comporte qui sont empilés les uns au-dessus des autres.

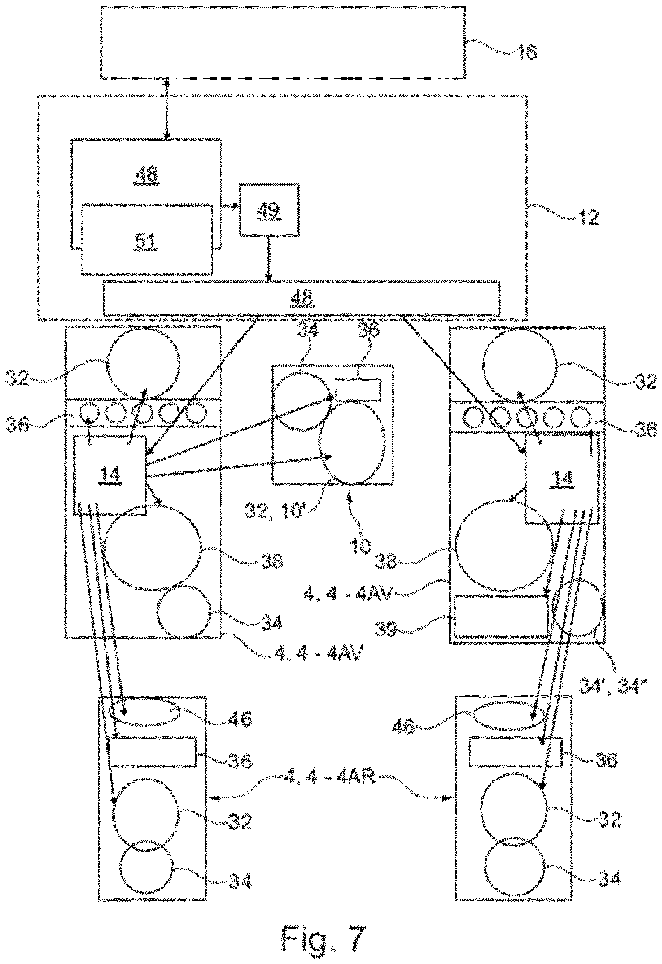

On va maintenant décrire plus en détails le pilotage des différents dispositifs sensoriels pour qu'ils émettent des effets sensoriels de façon synchronisée avec l'image projetée à l'écran et dans plusieurs endroits différents autour de l'utilisateur, afin de réaliser un effet immersif réussi, en se référant notamment à la figure 7.

Le dispositif immersif 1 comprend un module de commande principal 12 connecté au dispositif de projection 16 d'une part, et à l'ensemble des dispositifs sensoriels 8 d'autre part. Le module de commande principal 12 comprend un ordinateur 48 qui est d'une part relié au dispositif de projection 16 et qui est par ailleurs connecté à une interface de multiplexage numérique 50 de préférence de type DMX, par exemple via une connexion filaire 49 de type USB.

L'ordinateur 48 du module de commande principal 12 est implémenté avec une piste 51 modélisant la séquence d'images ou la vidéo projetée sur l'écran et associant à chaque instant de la dure totale de cette piste des effets sensoriels à produire en concordance avec l'image projetée. Le module de commande est ainsi apte à piloter l'ensemble des dispositifs sensoriels présents dans chacun des caissons multi sensoriels en fonction de l'image projetée sur l'écran.

L'interface de multiplexage numérique 50, ou encore répartiteur DMX, envoie simultanément à des récepteurs 52, ou contacteurs, DMX, respectivement embarqués dans chacun des caissons multi sensoriels 4, des instructions de commande générales I1 pour chacun des caissons présents dans le caisson correspondant.

Le récepteur ou contacteur 52 est implémenté dans un module de commande spécifique 14 à chaque caisson qui dispatche par la suite des instructions de commande spécifiques pour chacun des dispositifs sensoriels, et notamment des instructions de marche/arrêt de ces dispositifs.

Dans l'exemple illustré, des instructions de commande générales I1 sont envoyées simultanément aux caissons multi sensoriels avant 4-AV gauche et droit en direction du module de commande spécifique 14 dédié à chacun des caissons. Dans un deuxième temps, les modules de commande spécifiques 14 envoient des premières instructions de commande spécifiques I2 à chacun des dispositifs sensoriels 8 présents dans le caisson multi sensoriel avant 4-AV correspondant et des deuxièmes instructions de commande spécifiques I3 à chacun des dispositifs sensoriels 8 présent dans le caisson multi sensoriel arrière 4-AR le plus proche.

Par ailleurs l'un et/ou l'autre des modules de commande spécifique envoie des troisièmes instructions de commande spécifiques I4 à chacun des dispositifs sensoriels présents dans le caisson central 10.

De la sorte, on peut faire correspondre à une image donnée un effet sensoriel spécifique émis dans une zone spécifique.

Une variante de réalisation du dispositif immersif selon l'invention est illustrée sur la figure 8. Conformément à ce qui a été décrit précédemment, le dispositif immersif 1 comporte un écran courbe 2 et une pluralité de caissons multisensoriels 4 qui définissent une zone immersive 6. La zone immersive est délimitée notamment par l'écran courbe, et les caissons multisensoriels sont disposées en périphérie de la zone immersion. Les caissons multisensoriels principaux, ou caissons avant comme précédemment évoqués, sont disposés aux extrémités de part et d'autre de l'écran courbe, qui peut notamment consister en une toile enfilée autour d'une structure tubulaire courbe, ici non visible car recouverte par la toile.

Le dispositif immersif 1 diffère de ce qui précède en ce que l'écran courbe présente un profil d'arc de cercle d'environ 270° et en ce que les caissons multisensoriels en colonne sont ici au nombre de deux, agencés respectivement à chaque extrémité de l'écran courbe.

Un dispositif de projection 16 est associé spécifiquement à une portion de l'écran, ici s'étendant sur 90°, de sorte que trois dispositifs de protection sont ici prévus, chaque dispositif de projection étant configuré pour projeter sur sa portion d'écran associé une portion de l'image globale tel qu'elle doit être perçue au final par l'utilisateur.

Chaque dispositif de projection 16 est monté sur un portique 54 dont une barre centrale 56 s'étend au-dessus de la portion de l'écran associée. Plus particulièrement, le portique présente des montants 58, ici au nombre de quatre, fixés au sol et régulièrement répartis, ainsi que des barres centrales qui relient deux à deux les montants, de manière à ce que chaque barre centrale 56 s'étend au-dessus d'une des portions d'écran.

On comprend que l'on forme ainsi une pluralité de modules 100 présentant une portion d'écran avec un profil d'arc de cercle de 90°, avec outre cette portion d'écran courbe, des montants entre lesquels s'étend la barre centrale, et un dispositif de projection propre à chaque module.

De la sorte, il est possible de prévoir sans sortir du contexte de l'invention une zone immersive de dimension appropriée au besoin de l'utilisateur, en réalisant un dispositif immersif 1 par l'intermédiaire d'un ou plusieurs modules mis côte à côté, avec un module de commande 12 du système immersif qui est configuré pour piloter les dispositifs de projection et adapter la projection par chaque dispositif de projection de portions de l'image finale vue par l'utilisateur en fonction du nombre de modules mis en série.

Dans un dispositif immersif selon cette variante de réalisation, et notamment dans le cas d'exemple illustré d'un écran courbe à 270°, dans lequel un système de projection comporte plusieurs dispositifs de projection, le module de commande est configuré pour coordonner ces dispositifs de projection pour projeter une image continue le long de l'écran courbe, le cas échéant dans les zones de recouvrement d'images projetées d'un module à l'autre.

Chaque dispositif de projection comporte ici un vidéoprojecteur qui est disposé au-dessus de la zone immersive, le vidéoprojecteur 16 étant associé comme précédemment à un miroir 18 pour optimiser l'encombrement. Le vidéoprojecteur est fixé sur un carter 60 solidaire de la barre centrale du portique et le miroir est porté en bout de bras 62 s'étendant depuis le carter vers le centre de la zone immersive. La projection d'images par le vidéoprojecteur est réalisée dans un sens à l'opposé de l'écran et le miroir est agencé pour renvoyer l'image vers l'écran, depuis une position surplombant la zone immersive et à proximité de la portion d'écran qui lui est associé, de sorte que la présente d'utilisateur sensiblement au centre de la zone immersive ne gêne pas la projection d'images et ne nuit pas au caractère immersif de cette projection.

Le dispositif immersif comprend un caisson central 10 propre à chaque module et qui est dans l'exemple illustré porté par une barre 64 agencée en retrait de l'écran, au-dessus d'une zone de dégagement 66. Il convient de noter que ce caisson central pourrait être disposé à l'aplomb de l'écran sur la barre centrale, au voisinage du carter de support du projecteur.

La zone de dégagement 66 permet de loger des équipements électroniques, et notamment le module de commande avec le cas échéant une cloison 68 ici carrée, pour cacher ces zones de dégagement. La cloison 68 présente une ouverture 70 pour l'accès de l'utilisateur à la zone immersive 6.

On va maintenant décrire un exemple d'application du dispositif immersif selon l'invention, au cours duquel on vise à faire tester à un utilisateur un produit dans un environnement approprié, et par exemple une huile solaire dans un environnement représentatif d'une plage.

Comme cela est le cas pour la plupart des exemples d'application qui pourraient être cités, les caissons multi sensoriels sont commandés de manière à générer de façon quasi systématique une diffusion d'air et une diffusion de sons, et donc simultanément à la production de tout autre effet sensoriel. Le son est diffusé de manière continue, notamment pour couvrir les bruits de fonctionnement de chaque dispositif sensoriel. Et un flux d'air est diffusé aussi bien lorsque l'on souhaite faire ressentir à l'utilisateur l'existence de vent que lorsque l'on produit un effet olfactif, pour participer à la diffusion d'odeurs. La présence de ventilateurs formant dispositif de génération d'un flux d'air dans chacun des caissons permet de recréer de façon optimale la perception du vent arrivant de n'importe quel endroit de la zone immersive et permet d'améliorer une portance appropriée des effets de brumisation et des odeurs.

Lorsqu'une image projetée correspond à un palmier dont les feuilles bougent sous l'effet du vent, le module de commande envoie une instruction via l'interface DMX à chacun des dispositifs de génération d'un flux d'air, et le cas échéant aux dispositifs de génération d'un flux d'air additionnels, pour générer un flux d'air représentatif d'un coup de vent faisant bouger les feuilles.

Lorsque plus tard, une image projetée correspond à une vague formée sous l'effet d'un vent plus fort, le module de commande envoie via l'interface DMX des instructions à chacun des dispositifs de génération d'un flux d'air, et le cas échéant aux dispositifs de génération d'un flux d'air additionnels, pour générer un flux d'air représentatif de ce coup de vent plus fort et d'autres instructions à chacun des brumisateurs pour générer la formation de gouttelettes dispersées par le dispositif de génération d'un flux d'air afin que l'utilisateur ressente l'humidité formée par la vague.

L'utilisateur peut ainsi se sentir vraiment à la plage, dans la situation où il utilisera le produit à tester, de manière à être complètement impliqué dans le test du produit.

D'autres applications pourraient être mises en oeuvre et par exemple des applications ludiques ou des applications médicales.

## Revendications

1. Dispositif immersif (1) comportant un écran et une pluralité de caissons multi sensoriels (4) qui délimite avec l'écran une zone immersive, les différents caissons multi sensoriels étant disposés sur la périphérie de la zone immersive, chaque caisson multi sensoriels comportant au moins un dispositif de génération d'un flux d'air (32) et un dispositif générateur de son (34), ainsi qu'un dispositif de contrôle (12, 14) qui est configuré pour gérer l'émission de son et d'air par un pilotage indépendant de chacun des caissons multi sensoriels (4).

2. Dispositif immersif selon la revendication précédente, dans lequel au moins un caisson multi sensoriel (4) comporte au moins un dispositif de diffusion d'odeur (36), formé d'au moins un support de diffusion d'odeur (36') et un ventilateur (36"), et un dispositif de génération d'un flux d'air (32), le dispositif de diffusion d'odeur (36) et le dispositif de génération d'un flux d'air (32) étant disposés l'un au-dessus de l'autre.

3. Dispositif immersif selon l'une des revendications 1 ou 2, **caractérisé en ce qu'**il comporte un caisson central (10) agencé entre les au moins deux caissons multi sensoriels (4, 4-AV).

4. Dispositif immersif selon la revendication précédente, **caractérisé en ce que** le caisson central (10) comporte un ventilateur (10') configuré pour redistribuer l'air brassé par les dispositifs de génération d'un flux d'air (32) agencés dans chaque caisson multi sensoriel (4, 4-AV).

5. Dispositif immersif selon l'une des revendications précédentes, **caractérisé en ce que** l'écran (2) est courbe.

6. Dispositif immersif selon la revendication précédente, **caractérisé en ce que** les au moins deux caissons multi sensoriels comportent deux caissons multi sensoriels avants (4-AV) agencés respectivement dans le prolongement des extrémités de l'écran courbe (2).

7. Dispositif immersif selon l'une des revendications précédentes, en combinaison avec la revendication 3, **caractérisé en ce que** le caisson central (10) est disposé au centre et au-dessus de la zone immersive (6).

8. Dispositif immersif selon l'une des revendications précédentes, **caractérisé en ce que** l'écran (2) est associé à un dispositif de projection d'images (16).

9. Dispositif immersif selon la revendication précédente, **caractérisé en ce que** le dispositif de projection d'images (16) est un rétroprojecteur agencé du côté de l'écran (2) opposé à la zone immersive (6).

10. Dispositif immersif selon l'une des revendications précédentes, **caractérisé en ce qu'**il comporte deux caissons multi sensoriels dit avants (4-AV), agencés au voisinage direct de l'écran (2), deux caissons multi sensoriels dit arrière (4-AR), agencés à l'extrémité opposée de la zone immersive (6), et un caisson central (10) conforme à la revendication 3.

11. Dispositif immersif selon l'une des revendications précédentes, **caractérisé en ce que** le dispositif de contrôle (12, 14) est configuré pour répartir de façon équilibrée les effets entre chacun des caissons multi sensoriels (4) en fonction de l'image sur l'écran (2).

## Patentansprüche

1. Immersive Vorrichtung (1), die Folgendes umfasst: einen Bildschirm und eine Vielzahl von multisensorischen Gehäusen (4), die zusammen mit dem Bildschirm einen immersiven Bereich abgrenzt, wobei die unterschiedlichen multisensorischen Gehäuse am Umfang des immersiven Bereichs angeordnet sind, wobei jedes multisensorische Gehäuse mindestens eine Vorrichtung zur Erzeugung eines Luftstroms (32) und eine Vorrichtung zur Erzeugung von Klang (34) umfasst, sowie eine Steuerungsvorrichtung (12, 14), die dazu konfiguriert ist, die Emission von Klang und Luft durch eine unabhängige Ansteuerung jedes der multisensorischen Gehäuse (4) zu verwalten.

2. Immersive Vorrichtung nach dem vorhergehenden Anspruch, wobei mindestens ein multisensorisches Gehäuse (4) mindestens eine Vorrichtung zur Diffusion eines Geruchs (36), die durch mindestens einen Träger zur Diffusion eines Geruchs (36') und einen Ventilator (36'') gebildet wird, und eine Vorrichtung zur Erzeugung eines Luftstroms (32) umfasst, wobei die Vorrichtung zur Diffusion eines Geruchs (36) und die Vorrichtung zur Erzeugung eines Luftstroms (32) übereinander angeordnet sind.

3. Immersive Vorrichtung nach einem der Ansprüche 1 oder 2, **dadurch gekennzeichnet, dass** sie ein zentrales Gehäuse (10) umfasst, das zwischen den mindestens zwei multisensorischen Gehäusen (4, 4-AV) eingerichtet ist.

4. Immersive Vorrichtung nach dem vorhergehenden Anspruch, **dadurch gekennzeichnet, dass** das zentrale Gehäuse (10) einen Ventilator (10') umfasst, der dazu konfiguriert ist, die Luft, die durch die Vorrichtungen zur Erzeugung eines Luftstroms (32), die in jedem multisensorischen Gehäuse (4, 4-AV) eingerichtet sind, verwirbelt wird, weiterzuverteilen.

5. Immersive Vorrichtung nach einem der vorhergehenden Ansprüche, **dadurch gekennzeichnet, dass** der Bildschirm (2) gewölbt ist.

6. Immersive Vorrichtung nach dem vorhergehenden Anspruch, **dadurch gekennzeichnet, dass** die mindestens zwei multisensorischen Gehäuse zwei vordere multisensorische Gehäuse (4-AV) umfassen, die jeweils in der Verlängerung der Enden des gewölbten Bildschirms (2) eingerichtet sind.

7. Immersive Vorrichtung nach einem der vorhergehenden Ansprüche in Kombination mit Anspruch 3, **dadurch gekennzeichnet, dass** das zentrale Gehäuse (10) in der Mitte und über dem immersiven Bereich (6) angeordnet ist.

8. Immersive Vorrichtung nach einem der vorhergehenden Ansprüche, **dadurch gekennzeichnet, dass** der Bildschirm (2) mit einer Bildprojektionsvorrichtung (16) assoziiert ist.

9. Immersive Vorrichtung nach dem vorhergehenden Anspruch, **dadurch gekennzeichnet, dass** die Bildprojektionsvorrichtung (16) ein Tageslichtprojektor ist, der auf der dem immersiven Bereich (6) abgewandten Seite des Bildschirms (2) eingerichtet ist.

10. Immersive Vorrichtung nach einem der vorhergehenden Ansprüche, **dadurch gekennzeichnet, dass** sie zwei sogenannte vordere multisensorische Gehäuse (4-AV), die in direkter Nachbarschaft des Bildschirms (2) eingerichtet sind, zwei sogenannte hintere multisensorische Gehäuse (4-AR), die an dem entgegengesetzten Ende des immersiven Bereichs (6) eingerichtet sind, und ein zentrales Gehäuse (10) gemäß Anspruch 3 umfasst.

11. Immersive Vorrichtung nach einem der vorhergehenden Ansprüche, **dadurch gekennzeichnet, dass** die Steuerungsvorrichtung (12, 14) dazu konfiguriert ist, die Effekte in Abhängigkeit von dem Bild auf dem Bildschirm (2) ausgewogen zwischen jedem der multisensorischen Gehäuse (4) aufzuteilen.

## Claims

1. Immersive device (1) comprising a screen and a plurality of multi-sensory cases (4) which, with the screen, delimits an immersive zone, the various multi-sensory cases being arranged around the periphery of the immersive zone, each multi-sensory case comprising at least one air-flow generating device (32) and a sound-generating device (34), as well as a control device (12, 14) which is configured to manage the emission of sound and of air by independently controlling each of the multi-sensory cases (4).

2. Immersive device according to the preceding claim, in which at least one multi-sensory case (4) comprises at least one smell-diffusing device (36), formed of at least one smell-diffusing support (36') and a blower (36''), and an air-flow generating device (32), the smell-diffusing device (36) and the airflow generating device (32) being arranged one above the other.

3. Immersive device according to one of Claims 1 and 2, **characterized in that** it comprises a central case (10) arranged between the at least two multi-sensory cases (4, 4-AV).

4. Immersive device according to the preceding claim, **characterized in that** the central case (10) contains a blower (10') configured to redistribute the air agitated by the airflow generating devices (32) arranged in each multi-sensory case (4, 4-AV).

5. Immersive device according to one of the preceding claims, **characterized in that** the screen (2) is curved.

6. Immersive device according to the preceding claim, **characterized in that** the at least two multi-sensory cases comprise two front multi-sensory cases (4-AV) arranged respectively in the continuation of the ends of the curved screen (2).

7. Immersive device according to one of the preceding claims, in combination with Claim 3, **characterized in that** the central case (10) is arranged at the centre of and above the immersive zone (6).

8. Immersive device according to one of the preceding claims, **characterized in that** the screen (2) is associated with an image-projection device (16).

9. Immersive device according to the preceding claim, **characterized in that** the image-projection device (16) is a rear-projection device arranged on the opposite side of the screen (2) to the immersive zone (6).

10. Immersive device according to one of the preceding claims, **characterized in that** it comprises two multi-sensory cases referred to as front cases (4-AV), arranged in the direct vicinity of the screen (2), two multi-sensory cases referred to as rear cases (4-AR), arranged at the opposite end of the immersive zone (6), and a central case (10) according to Claim 3.

11. Immersive device according to one of the preceding claims, **characterized in that** the control device (12, 14) is configured to distribute the effects between each of the multi-sensory cases (4) in a balanced fashion according to the image on the screen (2).
